# DEMANDE DE BREVET EUROPEEN

(11) **EP 2 859 866 A1**
(43) Date de publication de la demande: **15.04.2015**
(21) Numéro de dépôt: 14188839.6
(22) Date de dépôt: 14.10.2014
(51) Int. Cl.: A61F 2/38, A61F 2/30

(54) **Implant tibial de prothèse de genou comportant une embase et un plateau support à épaisseur variable et procédé d'assemblage de ces derniers**

(30) Priorité: 14.10.2013 FR 1359994
(71) Demandeur: Euros, 13600 La Ciotat (FR)
(72) Inventeur: Limouzy, Frédéric, 31000 TOULOUSE (FR)
(74) Mandataire: Novagraaf Technologies

(57) **Abrégé**

Implant tibial d'une prothèse d'articulation de genou comportant un plateau support (1), une embase tibiale (2) et des moyens d'assemblage et de solidarisation du plateau (1) sur l'embase (2), comprenant une butée arrière (4) sur celle-ci, apte à immobiliser un rebord (5) arrière du plateau (1), un rebord avant (7) de celui-ci apte à se clipper dans une forme compatible (6) de l'embase (2), et des moyens de guidage disposés deux par deux latéralement de part et d'autre de la surface supérieure (172) de l'embase (2), ouverts au moins sur l'avant de celle-ci, et de part et d'autre de la surface inférieure (171) du plateau support (1), lesdits moyens de guidage (3, 13) étant aptes à coopérer entre eux lors du glissement avec contact continu du plateau support (1) sur et contre l'embase (2), et ladite forme compatible (6) étant disposée à une distance donnée sous la surface supérieure de contact (172) de l'embase (2), ledit rebord avant (7) du plateau support (1) étant lui-même décalé par rapport à la surface inférieure (17₁) du plateau support (1) de la même distance pour coopérer avec la dite forme compatible (6).

## Description

La présente invention a pour objet un implant tibial de prothèse de genou comportant une embase et un plateau support à épaisseur variable, ainsi qu'un procédé d'assemblage dudit plateau et de ladite embase d'un implant tibial de prothèse orthopédique destiné à équiper l'articulation d'un genou.

Le domaine de l'invention est celui des prothèses et dispositifs articulaires spécifiques aux genoux car ceux-ci nécessite des techniques particulières du fait de la complexité de l'articulation et des difficultés d'accès pour la mise en place de ces dispositifs par le chirurgien.

Cette articulation est en effet très sollicitée et peut ainsi subir des dégradations, en particulier au niveau des cartilages recouvrant le plateau tibial et le condyle fémoral suite à l'usure, à un accident mécanique et/ou dues au développement d'arthrose; il a donc été étudié et développé, depuis de nombreuses années, différents types de prothèses et dispositifs pour tenter de remédier à ces dégradations en remplaçant les surfaces naturelles abîmées par des implants artificiels, soit uni-compartimentaux qui ne concernent alors qu'un côté du genou, soit bi-compartimentaux.

Dans tous les cas, ces prothèses sont constituées du côté du fémur par un ou deux condyles dont la forme extérieure tend à se rapprocher de celle de la tête naturelle d'un fémur et du côté du tibia par un implant, dit alors tibial, comportant lui-même en général au moins deux parties assemblées entre elles: une partie, dite embase, qui est fixée dans la tête du tibia par tout moyen, tel que des vis passant à travers des orifices de l'embase, ladite tête de tibia étant coupée ou réséquée préalablement pour assurer un bon appui et une fixation de ladite embase, et une autre partie, dit plateau ou insert, qui vient se fixer sur l'embase et qui reçoit en appui le ou les condyles fémoraux, ceux-ci pouvant être considérés comme « roulant » sur ce plateau lors de la flexion et de l'extension de l'articulation.

La présente invention porte plus spécifiquement sur ce plateau et cette embase et leur procédé d'assemblage, pour lesquels on connaît différentes prothèses composées de tels implants qui ont fait l'objet de nombreux dépôts de demandes de brevet, telle que la demande US 5 344 460 de la société ENCORE ORTHOPEDICS INC, publiée le 6 septembre 1994 dans laquelle le plateau est inséré, par-dessus l'embase, à l'intérieur du rebord périphérique fermé en forme d'ovale de celui-ci, et qui est immobilisé par des formes périphériques en « L » qui s'emboîtent les unes dans les autres, complété par un vissage central et/ou du ciment.

Diverses autres demandes de brevet, telle que la demande FR 2 824 260 de la société BIOMET MERCK FRANCE, publiée le 8 novembre 2002 ou la demande internationale WO 2009/128943 de Monsieur Steven MANDELL, publiée le 22 octobre 2009 enseignent pour l'une des moyens d'immobilisation du plateau, dit patin, sur l'embase comprenant des moyens d'indexage, ainsi que de verrouillage par clavetage et d'ancrage avec au moins un crochet s'engageant dans une entaille, et pour l'autre un rail de guidage central sur l'embase coopérant avec une rainure du plateau et une immobilisation de celui-ci sur l'embase par des jeux de forme compatibles sur l'embase et le plateau, en avant et en arrière respectivement du rail de guidage et de la rainure.

Tous ces dispositifs, d'une part, ne sont adaptés qu'à des prothèses bi-compartimentales de genou et, d'autre part, nécessitent, lors de leur mise en place, d'écarter le condyle fémoral en soulevant la tête du fémur par rapport au tibia d'une hauteur significative qui tire alors exagérément sur les tendons et les ligaments du genou pour passer le plateau au-dessus de l'embase, afin de l'y emboîter et le fixer.

De plus, ces dispositifs d'assemblage connus imposent une hauteur assez grande de l'embase, ce qui diminue d'autant plus celle initiale du plateau, compte tenu de l'espace total disponible : ceci est défavorable car l'usure du plateau, par le frottement et l'appui des condyles du fémur, en diminue progressivement l'épaisseur, et quand celle-ci devient trop faible, il faut procéder à un remplacement du plateau, ce qui nécessite d'autant plus rapidement une nouvelle intervention que l'épaisseur du plateau est initialement déjà assez réduite.

On considérera dans la présente description les positions de dessus, dessous, inférieure, supérieure, avant, arrière ... des dispositifs et éléments de prothèse comme celles du genou sur lequel ils sont destinés à être fixés, lequel genou étant considéré en position debout.

Le problème posé est de remédier aux inconvénients des dispositifs connus à ce jour d'une part en réduisant au mieux la hauteur de l'embase pour augmenter le plus possible celle du plateau, dit insert, d'autre part en permettant une mise en place et un assemblage qui ne nécessitent pas de trop tirer sur les ligaments lorsqu'on soulève la tête de fémur par rapport à la tête du tibia, et également en proposant un dispositif d'assemblage qui soit adapté non seulement aux prothèses bi-compartimentales, mais aussi aux prothèses uni-compartimentales.

Une solution au problème posé est un implant tibial d'une prothèse d'articulation de genou, dont l'intersection du plan de rotation, ou dit plan sagittal, et du plan d'appui du condyle d'un implant fémoral sur ledit implant tibial, forme un axe définissant une direction de l'avant vers l'arrière de la prothèse, correspondant comme indiqué précédemment aux mêmes définitions de position que celle du genou à laquelle la prothèse est destinée ; lequel implant comporte :
- un plateau support apte à coopérer et à supporter l'appui d'au moins un condyle d'un implant fémoral,
- une embase tibiale pourvue d'au moins un moyen d'insertion apte à fixer ladite embase à l'extrémité supérieure d'un tibia réséqué, et
- des moyens d'assemblage et de solidarisation, l'un contre l'autre, du plateau sur l'embase, comprenant :
- une butée arrière sur celle-ci, apte à immobiliser un rebord arrière du plateau et
- un rebord avant de celui-ci apte à se clipper dans une forme compatible de l'embase, la surface dite inférieure du plateau étant alors posée et solidarisée sur la surface dite supérieure en vis-à-vis de l'embase.

Suivant l'invention les dits moyens d'assemblage et de solidarisation comprennent en outre :
- des moyens de guidage disposés deux par deux latéralement, de part et d'autre, c'est à dire de chaque côté dans le sens de la direction de l'avant vers l'arrière de la prothèse, d'une part de la surface supérieure de l'embase, ouverts au moins sur l'avant de celle-ci qui est ainsi dégagée de tout appendice, et définissant une direction de guidage parallèle à la direction de l'avant vers l'arrière de la prothèse, et d'autre part de la surface inférieure du plateau support, et lesdits moyens de guidage étant aptes à coopérer entre eux, d'une part lors du glissement, suivant la direction de guidage et avec contact continu du plateau support sur et contre l'embase, et, d'autre part, pour maintenir dans la position voulue d'assemblage du plateau support et de l'embase, par respectivement la butée et le rebord arrières et, le rebord avant du plateau support et la forme compatible de l'embase,
- laquelle forme compatible est disposée à une distance donnée sous la surface supérieure de contact de l'embase, ledit rebord avant du plateau support étant lui-même décalé, par rapport à la surface inférieure du plateau support, de la même distance pour coopérer avec la dite forme compatible.

Dans un mode particulier de réalisation suivant l'exemple de réalisation décrit plus précisément ci-après et représenté sur les figures jointes, le plateau support et l'embase sont ceux d'une prothèse uni-compartimentale du genou.

Dans des modes préférentiels, mais non limitatifs, de réalisation les moyens de guidage sont des glissières droites, parallèles entre elles et à la direction de l'avant vers l'arrière de la prothèse, et le rebord avant du plateau support est celui d'un bossage mâle formant une excroissance par rapport à la surface inférieure du plateau support, et la forme compatible de l'embase est celle au moins d'une partie de la périphérie d'un logement femelle formant un orifice réalisé dans la surface supérieure et vers l'extrémité avant de l'embase.

Une autre solution au problème posé est un procédé d'assemblage d'un plateau support d'un implant tibial d'une prothèse d'articulation de genou, apte à supporter l'appui d'au moins un condyle d'un implant fémoral et d'une embase tibiale pourvue de moyens d'insertion aptes à fixer ladite embase à l'extrémité supérieure d'un tibia réséqué, l'intersection du plan de rotation dit sagittal de ladite articulation et du plan d'appui du condyle de l'implant fémoral sur ledit implant tibial formant un axe définissant une direction de l'avant vers l'arrière de la prothèse, correspondant aux mêmes définitions de position que celles du genou à laquelle ladite prothèse est destinée, et tel que suivant l'invention:
- on fait glisser, suivant et dans la direction de l'avant vers l'arrière de la prothèse, ledit plateau support contre et sur l'embase,
- on assure le guidage du plateau support contre l'embase par des moyens de guidage disposés deux par deux latéralement, comme défini précédemment, et respectivement d'une part, de part et d'autre de la surface supérieure de l'embase, ouverts au moins sur l'avant de celle-ci qui est ainsi dégagée de tout appendice, et définissant une direction de guidage parallèle à la direction de l'avant vers l'arrière de la prothèse, et d'autre part, de part et d'autre de la surface inférieure du plateau support, et lesdits moyens de guidage étant aptes à coopérer entre eux,
- on maintient ledit plateau support en contact, au moins en un point du rebord de sa partie arrière et en un point de sa partie avant, contre la surface plane de l'embase pendant tout le glissement jusqu'à ce que le rebord arrière du plateau support vienne s'immobiliser contre une butée arrière de l'embase de forme compatible et apte à empêcher le rebord arrière du plateau support de se soulever de l'embase,
- on clippe alors, par déformation élastique, un rebord avant du plateau support dans une forme compatible de l'embase en pivotant le plateau support autour de la butée arrière et en ramenant ainsi la surface inférieure du plateau support contre la surface supérieure de l'embase.

De tels nouveaux dispositifs orthopédiques destinés à équiper l'articulation d'un genou, et nouveaux procédés d'assemblage de leur plateau support d'implant tibial avec leur embase tibiale, permettent d'atteindre les objectifs cités précédemment et répondent ainsi au problème posé car :
- d'une part le décalage à un niveau inférieur des éléments de fixation avant par rapport à la surface supérieure de l'embase et
- d'autre part l'absence d'appendice et de rebord périphérique de l'embase sur son coté avant, puisque les guides latéraux sont ouverts de ce côté,
dégagent complètement et au mieux l'accessibilité par ce côté avant par lequel on peut glisser ainsi le plateau sur ladite embase au plus près de celle-ci, ce qui permet de diminuer la hauteur totale de l'implant lors de sa mise en place et donc limite l'amplitude du soulèvement de la tête de fémur sans avoir alors à trop tirer sur les ligaments.

En effet lors de la présentation de mise en place dans l'articulation puis du glissement du plateau support sur et contre l'embase, les caractéristiques de l'implant tibial suivant l'invention et telles que définies ci-dessus et ci-après permettent de maintenir les deux dites surfaces, respectivement inférieure du plateau support et supérieure de l'embase, en vis-à-vis suivant un angle mécanique α pouvant ne faire que 15° avec même une valeur minimum allant jusqu'à 5°, soit un angle mécanique compris entre 5 et 15°.

De plus, le guidage latéral et la disposition des éléments de fixation, à l'avant et à l'arrière, entre le plateau et l'embase, permettent de diminuer la hauteur h₂ de celle-ci et donc d'augmenter l'épaisseur h₁ utile du plateau, ce qui le rend plus rigide et efficace dans le temps, car il résistera ainsi plus longtemps à l'usure.

De tels nouveaux dispositifs et procédés permettent également de bien s'adapter aux prothèses uni-compartimentales, qui sont du reste représentées dans l'exemple décrit ci-après et ci-joint sur les figures jointes, mais peuvent également s'adapter à des prothèses bi-compartimentales.

Le résultat est un nouveau dispositif orthopédique et un nouveau procédé d'assemblage d'un plateau support sur une embase, en particulier pour ce qui concerne l'implant tibial destine à équiper l'articulation d'un genou dont les avantages évoqués ci-dessus en montrent l'intérêt et dont la description et les figures jointes en donnent un exemple de réalisation.

D'autres modes de réalisations sont cependant possibles dans le cadre de la portée de la présente invention.

La figure 1 représente, en vue perspective de côté et de dessus, un plateau support 1, ou dit aussi insert ou plateau intermédiaire, et une embase 2, ou dite aussi plaque tibiale, d'un implant tibial suivant l'invention, séparés l'un de l'autre, comme posés cote a cote sur une surface horizontale, le plateau support 1 étant retourné avec sa surface inférieure 17₁ dirigée vers le haut, lesquels plateau support 1 et embase 2 étant ceux d'une prothèse uni-compartimentale du genou.

La figure 2 représente les mêmes éléments que la figure 1 mais dans leur position d'assemblage, la surface 11, apte à recevoir en appui le condyle fémoral, orientée vers le haut.

La figure 3 représente le plateau support 1 de la figure 1 en vue perspective de côté et de dessous.

Les figures 4, 5 et 6 sont des vues en coupe, suivant un plan vertical médian d'un implant tibial suivant l'invention et parallèle à la direction de guidage 18, d'un plateau support 1 et d'une embase 2, tels que représentés sur les figures 1 à 3, en cours d'assemblage par glissement et contact de l'un sur l'autre respectivement :
- depuis une position d'écartement mécanique qui peut correspondre à un angle α de 15° et qui peut, grâce aux caractéristiques de l'invention, ne faire que 5° à 6° maximum, permettant de réduire l'espace nécessaire, entre les têtes de fémur et de tibia, pour la mise en place de l'implant, ce qui évite de trop tirer sur les ligaments mais bien sûr le chirurgien peut ouvrir d'avantage cet espace s'il le souhaite et effectuer le glissement du plateau sur l'embase avec un angle supérieur à ces 5 à 6°, qui est en fait alors pour lui un angle minimum de mise en oeuvre,
- puis une position de butée arrière et de pivotement du plateau support 1 pour rapprocher la surface inférieure 17₁ de celui-ci vers la surface supérieure 17₂ de l'embase 2 jusqu'à la position de clippage et d'assemblage finale.

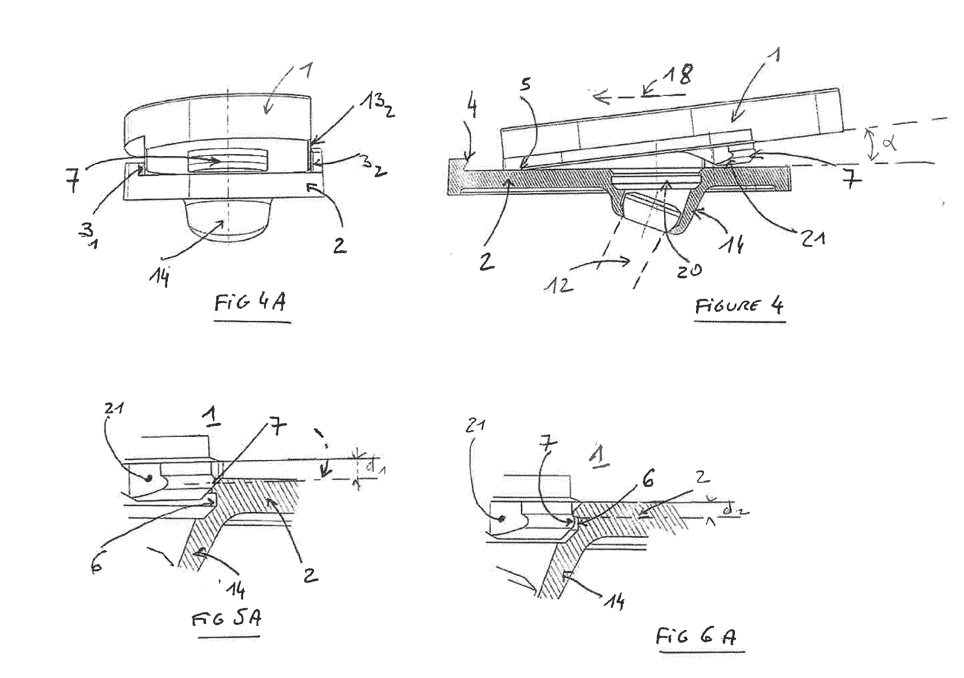

La figure 4A est une vue de l'avant d'un plateau support 1 et d'une embase 2 suivant l'invention, soit vue depuis la droite de la figure 4.

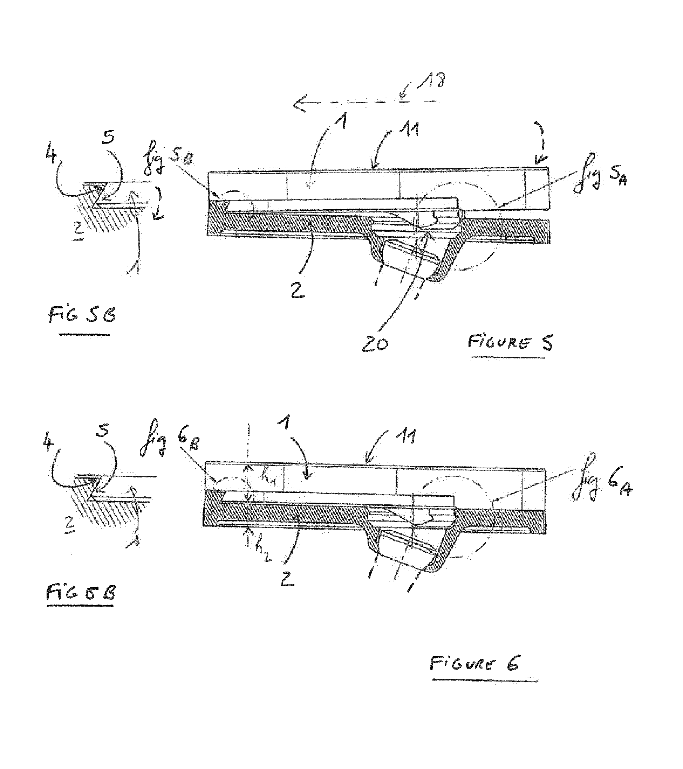

Les figures 5A, 5B, 6A et 6B sont des vues agrandies des détails entourés par des cercles de repérage sur les figures 5 et 6.

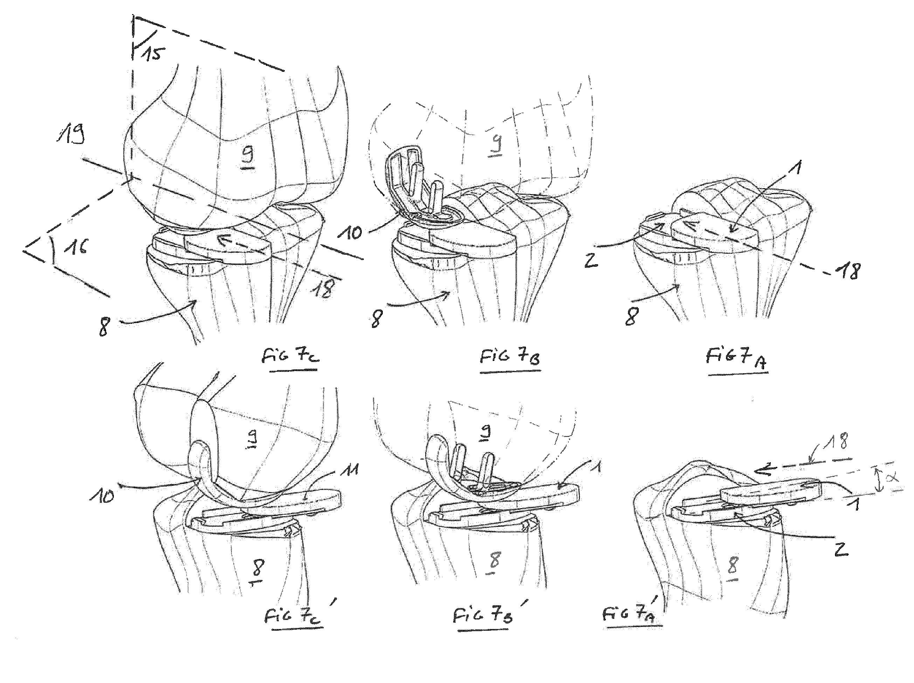

La figure 7A est une vue perspective de trois quart avant et de dessus de l'implant tibial suivant l'invention en cours d'assemblage par glissement de direction 18 du plateau support 1 sur l'embase 2 dans une position d'écartement maximum comme sur la figure 4, la dite embase 2 étant fixée sur l'extrémité supérieure 8 d'un tibia réséquée.

Les figures 7B et 7C représentent les mêmes éléments que la figure 7A mais avec un condyle fémoral 10 positionné sur la tête de fémur 9 du genou considéré, représentée respectivement en transparence et en vue extérieure, et écartée de l'implant tibial pour permettre le glissement du plateau support 1 suivant l'invention.

Les figures 7A', 7B' et 7C' représentent les mêmes éléments et dans la même position que sur les figures 7A, 7B et 7C, mais en vue perspective de trois quart arrière et de dessus de l'implant tibial suivant l'invention.

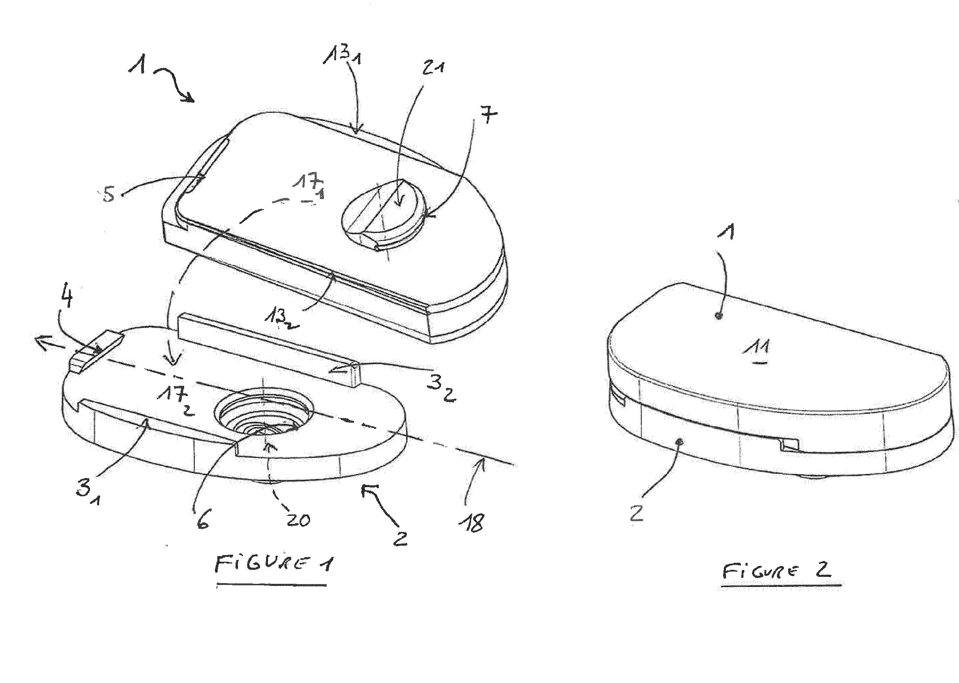

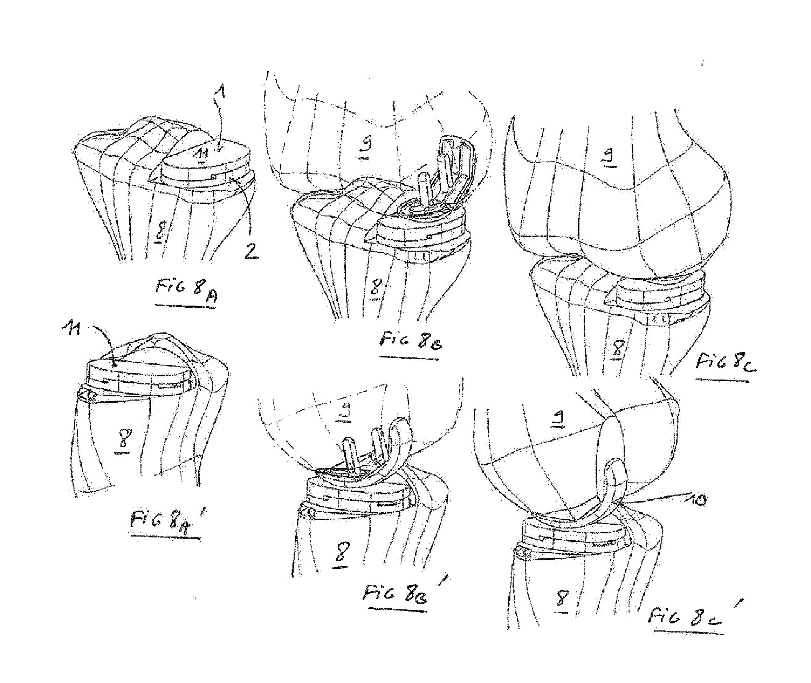

Les six figures 8A à 8C' représentent les mêmes éléments, et suivant les mêmes vues perspectives, respectivement que sur les six figures 7A' a 7C', mais en fin d'assemblage par clippage du plateau support 1 sur l'embase 2 comme sur les figures 2 et 6, la tête du fémur 9 reposant sur la surface d'appui supérieure 11 du plateau support 1 de l'implant tibial.

On définit comme direction 18 de l'avant vers l'arrière d'un implant tibial d'une prothèse d'articulation de genou, correspondant aux mêmes définitions de position que celle du genou à laquelle la prothèse est destinée, l'axe 19 forme par l'intersection du plan de rotation, ou dit plan sagittal 15, et du plan 16 d'appui du condyle 10 d'un implant fémoral sur ledit implant tibial.

Le dit implant tibial comporte, d'une manière connue :
- un plateau support 1 apte à coopérer et à supporter l'appui du au moins condyle de l'implant fémoral correspondant,
- une embase tibiale 2 pourvue d'au moins un moyen d'insertion 14 apte à fixer ladite embase a l'extrémité supérieure du tibia reséqué 8 de l'articulation du genou considérée, et
- des moyens d'assemblage et de solidarisation, l'un contre l'autre, du plateau 1 sur l'embase 2, comprenant une butée arrière 4 sur celle-ci, apte à immobiliser un rebord 5 arrière du plateau 1 et un rebord avant 7 de celui-ci apte à se clipper dans une forme compatible 6 de l'embase 2, la surface dite inférieure 17₁ du plateau 1 étant posée et solidarisée sur la surface 17₂ dite supérieure en vis-à-vis de l'embase 2.
   selon l'invention les moyens d'assemblage et de solidarisation comprennent en outre des moyens de guidage disposés deux par deux latéralement, c'est à dire de chaque côté dans le sens de la direction de l'avant vers l'arrière de la prothèse:
- d'une part 3, de part et d'autre de la surface supérieure 17₂, de préférence plane, de l'embase 2, ouverts au moins sur l'avant de celle-ci qui est ainsi dégagée de tout appendice, et définissant une direction de guidage parallèle à la direction 18, et
- d'autre part 13, de part et d'autre de la surface inférieure 17₁, de préférence plane également, du plateau support 1,
ce qui fait, comme représenté sur les figures, quatre moyens de guidage quand il y en a un par côté du plateau et de l'embase.

Lesdits moyens de guidage 3, 13 sont aptes à coopérer entre eux, d'une part, lors du glissement, suivant la direction 18 et avec contact continu du plateau support 1 sur et contre l'embase 2, et, d'autre part, pour maintenir dans la position voulue d'assemblage du plateau support 1 et de l'embase 2 par respectivement la butée 4 et le rebord 5 arrières et, le rebord avant 7 du plateau support 1 et la forme compatible 6 de l'embase 2.

De préférence ces moyens de guidage 3, 13 sont des glissières droites, parallèles entre elles et à la direction 18 d'axe 19, mais dans un autre mode de réalisation ces dits moyens de guidage 3, 13 peuvent être des glissières droites, disposées en tronc de cône convergeant de l'avant vers l'arrière, ledit tronc de cône étant d'axe de direction 18 : dans tous les cas les formes en coupe de ces glissières respectivement du plateau support 1 et de l'embase tibiale 2 sont telles qu'elles coopèrent entre elles :
- d'une part par leurs profils se faisant face lors du glissement, comme tout homme du métier sait le définir, et lors de l'assemblage, et
- d'autre part par les faces extérieures latérales des glissières de l'embase tibiale, une fois le clippage réalisé, et les faces des bords périphériques latéraux du plateau support 1, lesquelles faces doivent former entre elles sur chaque côté de l'implant tibial des surfaces continues latérales sans aspérité.

La dite forme compatible 6 est disposée à une distance donnée d₂ sous la surface supérieure de contact 17₂ de l'embase 2, et donc décalé à un niveau en dessous de celui de cette surface, ledit rebord avant 7 du plateau support 1 étant lui-même décalé, par rapport à la surface inférieure 17₁ du plateau support 1 dont il forme un appendice dépassant de celle-ci, d'une distance d₁ de même valeur pour coopérer avec la dite forme compatible 6.

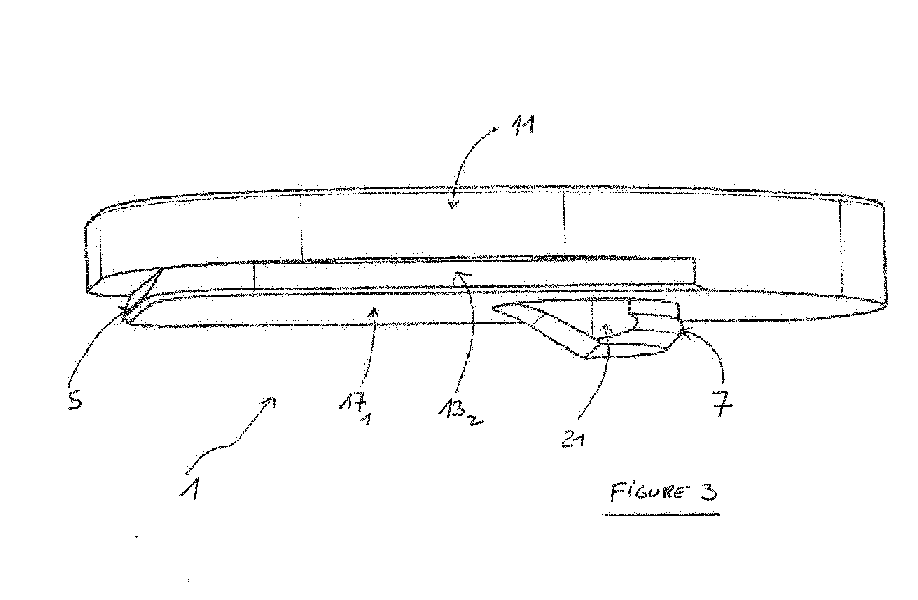

De préférence comme représenté sur les figures 1, 3, 4, 5 et 6, ce rebord avant 7 du plateau 1 est celui d'un bossage mâle 2₁ formant une excroissance par rapport à la surface inférieure 17₁ du plateau support 1 et la forme compatible 6 de l'embase 2 est celle au moins d'une partie de la périphérie d'un logement femelle formant un orifice 20 réalisé dans la surface supérieure et vers l'extrémité avant de l'embase 2, et le dit orifice 20 pouvant être celui d'un moyen d'insertion 14 apte à fixer ladite embase 2 à l'extrémité supérieure du tibia reséqué 8.

Suivant l'exemple de réalisation des figures 4 à 6, et plus précisément sur les figures 5B et 6B, la butée arrière 4 de l'embase 2 et le rebord 5 arrière du plateau 1 sont en forme de queues d'arondes formant respectivement une jonction mâle et femelle ; celles-ci coopèrent en effet entre elles d'une part pour assurer la fonction de butée arrière à la fin du glissement du plateau 1 contre l'embase 2 suivant la direction 18, puis d'autre part permettre la rotation par pivotement du plateau support 1 autour de cette butée arrière 4 et enfin l'immobilisation et donc l'assemblage du plateau support 1 et de l'embase 2 en empêchant le rebord arrière 5 du plateau 1 de se soulever par rapport à l'embase 2.

Grace aux caractéristiques de l'implant tibial suivant l'invention, on, c'est à dire en fait le chirurgien, peut, plus facilement qu'avec les implants existants précédemment, choisir en per opératoire le plateau support 1 d'épaisseur h₁ voulue en fonction de la distance souhaitée entre l'embase 2 et le condyle fémoral 10 pour rétablir au mieux l'équilibre des tensions articulaires du genou du patient ; de plus on, soit le chirurgien, peut modifier cette épaisseur à la demande en démontant et remplaçant facilement ledit plateau 1 par un autre, en per opératoire ou lors d'une nouvelle opération postérieure telle qu'en cas d'usure trop importante du plateau nécessitant son remplacement.

## Revendications

1. Implant tibial d'une prothèse d'articulation de genou, dont l'intersection du plan de rotation, ou dit plan sagittal (15), et du plan (16) d'appui du condyle d'un implant fémoral, sur ledit implant tibial, forme un axe (19) définissant une direction (18) de l'avant vers l'arrière de la prothèse correspondant aux mêmes définitions de position que celle du genou à laquelle la prothèse est destinée, lequel implant comporte un plateau support (1) apte à coopérer et à supporter l'appui d'au moins un condyle d'un implant fémoral, une embase tibiale (2) pourvue d'au moins un moyen d'insertion (14) apte à fixer ladite embase à l'extrémité supérieure d'un tibia reséqué, et des moyens d'assemblage et de solidarisation, l'un contre l'autre, du plateau (1) sur l'embase (2), comprenant une butée arrière (4) sur celle-ci, apte à immobiliser un rebord (5) arrière du plateau (1) et un rebord avant (7) de celui-ci apte à se clipper dans une forme compatible (6) de l'embase (2), la surface dite inférieure (17₁) du plateau (1) étant posée et solidarisée sur la surface (17₂) dite supérieure en vis-à-vis de l'embase (2), **caractérisé en ce que** les moyens d'assemblage et de solidarisation comprennent en outre :
- des moyens de guidage disposés deux par deux latéralement d'une part (3), de part et d'autre de la surface supérieure (17₂) de l'embase (2), ouverts au moins sur l'avant de celle-ci qui est ainsi dégagée de tout appendice, et définissant une direction de guidage parallèle à la direction (18), et d'autre part, de part et d'autre de la surface inférieure (17₁) du plateau support (1), et lesdits moyens de guidage (3, 13) étant aptes à coopérer entre eux, d'une part, lors du glissement, suivant la direction (18) et avec contact continu du plateau support (1) sur et contre l'embase (2), et, d'autre part, pour maintenir dans la position voulue d'assemblage du plateau support (1) et de l'embase (2) par respectivement la butée (4) et le rebord (5) arrières et, le rebord avant (7) du plateau support (1) et la forme compatible (6) de l'embase (2),
- laquelle forme compatible (6) est disposée à une distance donnée (d) sous la surface supérieure de contact (17₂) de l'embase (2), ledit rebord avant (7) du plateau support (1) étant lui-même décalé par rapport à la surface inférieure (17₁) du plateau support (1) de la même distance (d) pour coopérer avec la dite forme compatible (6).

2. Implant tibial d'une prothèse d'articulation de genou suivant la revendication 1, **caractérisé en ce que** les moyens de guidage (3, 13) sont des glissières droites, parallèles entre elles et à la direction (18) d'axe (19).

3. Implant tibial d'une prothèse d'articulation de genou suivant la revendication 1, **caractérisé en ce que** lesdits moyens de guidage (3, 13) sont des glissières droites, disposées en tronc de cône convergeant de l'avant vers l'arrière et respectivement du plateau support (1) et de l'embase tibiale (2), ledit tronc de cône étant d'axe de direction (18).

4. Implant tibial suivant l'une quelconque des revendications 1 à 3, **caractérisé en ce que** le rebord avant (7) du plateau (1) est celui d'un bossage mâle (21) formant une excroissance par rapport à la surface inférieure (17₁) du plateau support (1) et la forme compatible (6) de l'embase (2) est celle au moins d'une partie de la périphérie d'un logement femelle formant un orifice réalisé dans la surface supérieure et vers l'extrémité avant de l'embase (2).

5. Implant tibial suivant la revendication 4, **caractérisé en ce que** l'orifice (20) portant la forme compatible (6) de l'embase (2) est celui d'un moyen d'insertion (14) apte à fixer ladite embase (2) à l'extrémité supérieure du tibia reséqué.

6. Implant tibial suivant l'une quelconque des revendications 1 à 5, **caractérisé en ce que** la butée arrière (4) de l'embase (2) et le rebord (5) arrière du plateau (1) sont en forme de queues d'arondes formant respectivement une jonction mâle et femelle et coopérant entre elles.

7. Implant tibial suivant l'une quelconque des revendications 1 à 6, **caractérisé en ce que** le plateau support (1) et l'embase (2) sont ceux d'une prothèse uni-compartimentale du genou.

8. Implant tibial suivant l'une quelconque des revendications 1 à 7, **caractérisé en ce que** l'assemblage du plateau support (1) sur l'embase (2) se fait par glissement du plateau (1) contre l'embase (2) suivant et dans la direction (18), ledit plateau (1) restant en contact au moins en un point du rebord de sa partie arrière et en un point de sa partie avant contre la surface plane supérieure (17₂)de l'embase (2) pendant tout le glissement jusqu'à ce que le rebord (5) arrière du plateau (1) vienne s'immobiliser contre la butée arrière (4) de l'embase (2) apte à empêcher le rebord arrière (5) du plateau de se soulever de l'embase (2).

9. Implant tibial suivant la revendication 8 **caractérisé en ce que** lors du glissement du plateau support (1) sur et contre l'embase (2), la surface inférieure (17₁) du plateau support (1) et la surface plane supérieure (17₂) de l'embase (2) forment un angle mécanique α minimum allant jusqu'à 5°.

10. Implant tibial suivant une quelconque des revendications 1 à 9 **caractérisé en ce que** l'épaisseur (h₁) du plateau (1) est choisie en fonction de la distance souhaitée entre l'embase (2) et le condyle fémoral (10).
